# EUROPEAN PATENT SPECIFICATION

(11) **EP 2 540 829 B1**
(45) Date of publication and mention of the grant of the patent: **25.11.2015**
(21) Application number: 11747512.9
(22) Date of filing: 25.02.2011
(51) Int. Cl.: C12N 15/113, C12N 15/09, C12Q 1/68

(54) **MARKER FOR DETECTION OF MYOGENIC DISEASES, AND METHOD FOR DETECTION OF THE DISEASES USING SAME**
MARKER ZUR ERKENNUNG MYOGENER ERKRANKUNGEN UND VERFAHREN ZUR ERKENNUNG SOLCHER ERKRANKUNGEN MITHILFE DIESER MARKER
MARQUEUR POUR LA DÉTECTION DE MALADIES MYOGÈNES ET PROCÉDÉ DE DÉTECTION DE CES MALADIES L'UTILISANT

(30) Priority: 26.02.2010 JP 2010041845
(43) Date of publication of application: 02.01.2013
(73) Proprietor: LSIP, LLC, Chiyoda-ku Tokyo 100-0005 (JP)
(72) Inventor: HASHIDO Kazuo, Kodaira-shi Tokyo 187-8551 (JP); MIZUNO Hideya, Kodaira-shi Tokyo 187-8551 (JP)
(74) Representative: Cabinet Plasseraud
(86) International application number: PCT/JP2011/054318
(87) International publication number: WO 2011/105556

(56) References cited:
- WO-A1-2010/136415
- WO-A2-2009/134710
- JP-A- 11 313 682
- JP-A- 2005 304 481
- GRECO SIMONA ET AL: "Common micro-RNA signature in skeletal muscle damage and regeneration induced by Duchenne muscular dystrophy and acute ischemia", FASEB JOURNAL, FED. OF AMERICAN SOC. FOR EXPERIMENTAL BIOLOGY, US, vol. 23, no. 10, 1 October 2009 (2009-10-01), pages 3335-3346, XP002594658, ISSN: 0892-6638, DOI: 10.1096/FJ.08-128579
- CALLIS THOMAS E ET AL: "Muscling through the microRNA world", EXPERIMENTAL BIOLOGY AND MEDICINE, SOCIETY FOR EXPERIMENTAL BIOLOGY AND MEDICINE, US, vol. 233, no. 2, 1 February 2008 (2008-02-01), pages 131-138, XP002532418, ISSN: 1535-3702, DOI: 10.3181/0709-MR-237
- MCCARTHY J.J. ET AL: 'MicroRNA-206 is overexpressed in the diaphragm but not the hindlimb muscle of mdx mouse' AM J PHYSIOL CELL PHYSIOL vol. 293, 2007, pages C451 - C457
- CALLIS T.E. ET AL: 'Muscling through the microRNA world' EXPERIMENTAL BIOLOGY AND MEDICINE vol. 233, no. 2, 2008, pages 131 - 138
- EISENBERG I. ET AL: 'miRNAs in normal and diseased skeletal muscle' J. CELL. MOL. MED. vol. 13, no. 1, 2009, pages 2 - 11
- GRECO S. ET AL: 'Common micro-RNA signature in skeletal muscle damage and regeneration induced by duchenne muscular dystrophy and acute ischemia' THE FASEB JOURNAL vol. 23, October 2009, pages 3335 - 3346, XP002594658
- ARASHIRO P. ET AL: 'Transcriptional regulation differs in affected facioscapulohumeral muscular dystrophy patients compared to asymptomatic related carriers' PNAS vol. 106, no. 15, April 2009, pages 6220 - 6225
- EISENBERG I. ET AL: 'Distinctive patterns of microRNA expression in primary muscular disorders' PNAS vol. 104, no. 43, October 2007, pages 17016 - 17021
- Davide Cacchiarelli ET AL: "miRNAs as serum biomarkers for Duchenne muscular dystrophy", EMBO Molecular Medicine, vol. 3, no. 5, 21 March 2011 (2011-03-21), pages 258-265, XP055036012, ISSN: 1757-4676, DOI: 10.1002/emmm.201100133
- Hideya Mizuno ET AL: "Identification of Muscle-Specific MicroRNAs in Serum of Muscular Dystrophy Animal Models: Promising Novel Blood-Based Markers for Muscular Dystrophy", PLoS ONE, vol. 6, no. 3, 30 March 2011 (2011-03-30), page e18388, XP055036013, DOI: 10.1371/journal.pone.0018388

## Description

### Technical Field

The present invention relates to a marker for detecting myogenic disease and a method for detecting the presence or absence of myogenic disease, particularly, muscular dystrophy, affecting a test subject using the same.

### Background Art

Muscular dystrophy, a type of myogenic disease, is a progressive genetic disease that causes muscle wasting or weakness resulting from the degeneration or necrosis of muscle fibers in skeletal muscles. This disease is known to have various types, such as Duchenne, Becker, limb-girdle, and facioscapulohumeral types, depending on the mode of inheritance or clinical conditions (Non Patent Literature 1).

Muscular dystrophy is comprehensively diagnosed by means of clinical conditions, blood test, examination findings, electromyography, muscle biopsy, genetic test, etc. Of them, the blood test is conducted by the determination of the amount of an enzyme such as creatine kinase, lactate dehydrogenase, glutamic oxaloacetic transaminase (GOT), glutamic pyruvic transaminase (GPT), or aldolase.

The blood test method is based on the phenomenon in which these enzymes contained abundantly in myocytes, leak into blood due to myocyte necrosis in muscular dystrophy patients and thereby exhibit a high level compared with the normal state. This method is low invasive to test subjects because of allowing detection using peripheral blood. Also, its measurement procedures are relatively convenient. For these reasons, the method is widely used in the diagnosis of muscular dystrophy. Among others, a general method involves determining the serum level of creatine kinase (Non Patent Literature 2). However, the enzymes including creatine kinase also leak into blood by myocyte necrosis attributed to exercise stress. Their serum concentrations thus largely vary depending on the presence or absence of exercise stress to test subjects. As a result, even normal individuals exhibit a high level of creatine kinase or the like and are disadvantageously misdiagnosed. For accurate diagnosis, test subjects must be placed in the resting state before blood collection, and this process is burdensome.

### Citation List

### Non Patent Literature

Non Patent Literature 1: Hideo Sugita, Eijiro Ozawa, and Ikuya Nonaka, ed., 1995, Principle of Myology, Nankodo Co., Ltd., Tokyo, Japan: pp. 469-550
Non Patent Literature 2: Sugita H., et al., 1959, J. Biochem., 46: 103-104

### Summary of Invention

### Technical Problem

An object of the present invention is to provide a marker for detecting myogenic disease that is low invasive to test subjects and highly safe against exercise stress and a method for detecting myogenic disease, particularly, muscular dystrophy, using the same.

### Solution to Problem

As a result of conducting diligent studies to attain the object, the present inventors have found that the amounts of three types of micro-RNAs (miRNAs) including variants, i.e., miR-1, miR-133 (including two variants miR-133a and miR-133b), and miR-206, in blood are closely associated with myogenic disease, particularly, muscular dystrophy, affecting a test subject and are hardly susceptible to exercise stress. It has heretofore been known that: miR-1 and miR-133 are specifically expressed in cardiac muscles, atria, and skeletal muscles; and miR-206 is specifically expressed in skeletal muscles (Kim et al., 2006, J. Cell Biochem, 174, 677-687). Nevertheless, it has been totally unknown that the amounts of these miRNAs in blood can serve, in place of creatine kinase, as a useful marker for detection hardly susceptible to exercise stress for myogenic disease-affected individuals. The present invention has been completed based on these findings and provides the following aspects:
(1) A method for detecting the presence or absence of myogenic disease affecting a test subject, comprising the steps of: measuring the amount of one or more miRNAs comprising any of the nucleotide sequences shown in SEQ ID NOs: 1 to 4 in blood collected from the test subject; and relating the statistically significantly higher amount of the miRNA in the blood of the test subject than that of corresponding miRNA in the blood of a normal individual with the presence of myogenic disease affecting the test subject.
(2) The method according to (1), wherein each of the miRNA consists of one of the nucleotide sequences shown in SEQ ID NOs: 1 to 4.
(3) The method according to (1) or (2), wherein the amount of the miRNA in the blood of the test subject is 5 or more times that of corresponding miRNA in the blood of a normal individual.
(4) The method according to any of (1) to (3), wherein the amount of the miRNA in the blood is quantitated by a nucleic acid amplification method or a hybridization method.
(5) The method according to (4), wherein the nucleic acid amplification method is real-time PCR.
(6) The method according to any of (1) to (5), wherein the blood has been collected from the test subject after exercise stress.
(7) The method according to any of (1) to (6), wherein the myogenic disease is muscular dystrophy.
(8) A marker for detecting myogenic disease consisting of miRNA each of which comprises one of the nucleotide sequences shown in SEQ ID NOs: 1 to 4.

The present specification cites the contents described in the specification and/or drawings of Japanese Patent Application No. 2010-041845 which serves as a basis for the priority of the present application.

### Advantageous Effects of Invention

According to a marker for detecting myogenic disease of the present invention, there can be provided a marker for detecting myogenic disease affecting a test subject without being influenced by exercise stress.

According to a method for detecting myogenic disease of the present invention, there can be provided a method capable of detecting the presence or absence of myogenic disease affecting a test subject, wherein the method is low invasive to the test subject and is insusceptible to exercise stress to the test subject.

### Brief Description of Drawings

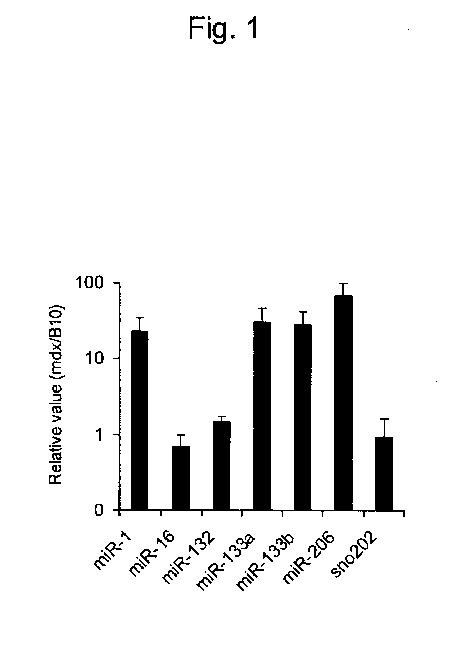

[Figure 1] Figure 1 shows the relative value of the amount of each miRNA in the serum of mdx mice to that in B10 mice.

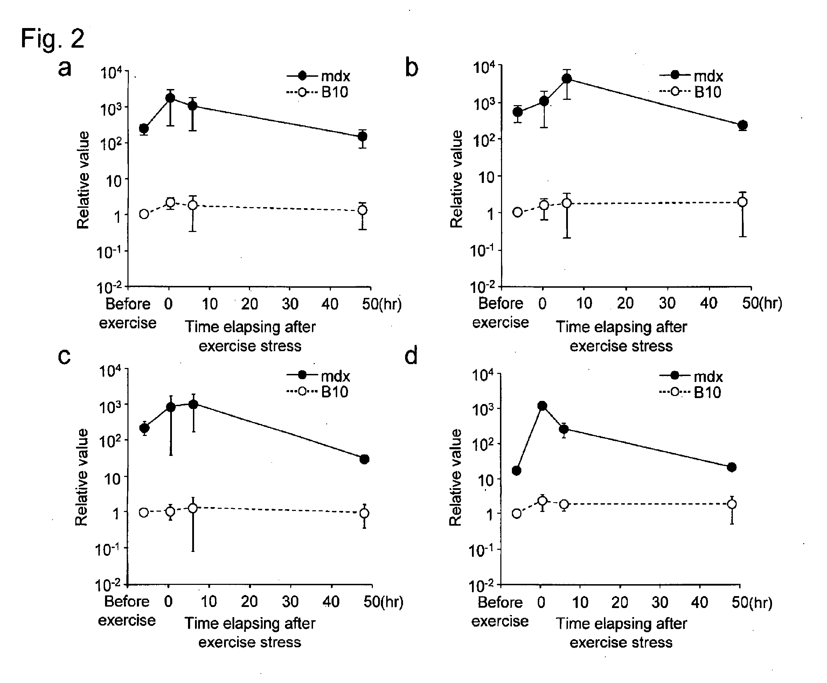

[Figure 2] Figure 2 is a diagram showing change in the amounts of miR-1, miR-133a, and miR-206 in the serum of B10 and mdx mice over time elapsing after exercise stress. The amounts of miR-1, miR-133a, and miR-206 in each mouse serum before exercise and at each point in time elapsing after exercise stress were corrected with the amount of miR-16 and then indicated by relative values to the corrected levels of the B10 mice before exercise. In the diagram, a represents miR-1; b represents miR-133a; c represents miR-206; and d represents creatine kinase (CK). The open circle/broken line represents B10 mice, and the filled circle/solid line represents mdx mice.

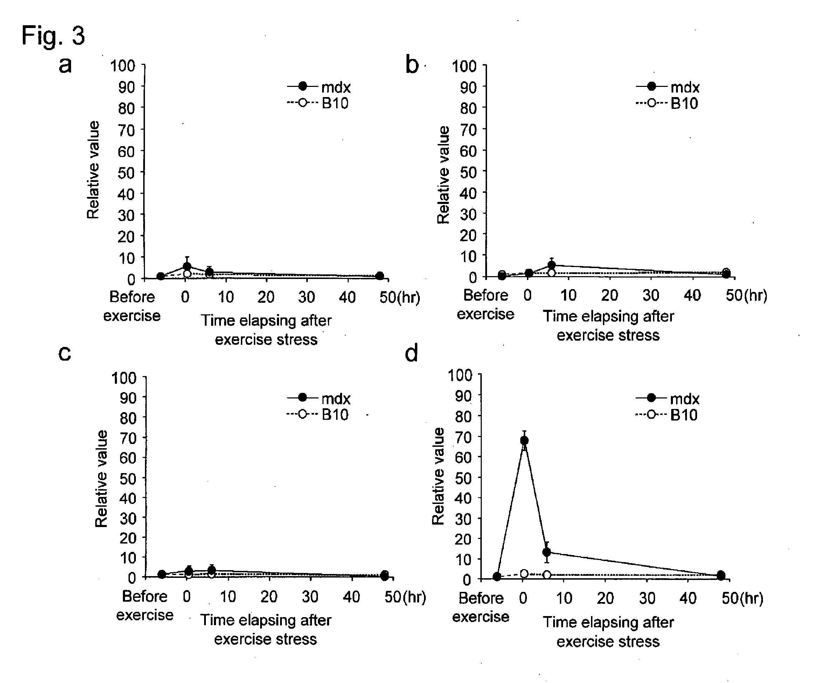

[Figure 3] Figure 3 is a diagram showing change in the amounts of miR-1, miR-133a, and miR-206 in the serum of B10 and mdx mice over time elapsing after exercise stress. The amounts of serum creatine kinase (CK), miR-1, miR-133a, and miR-206 were corrected with the amount of miR-16 and then indicated by relative values of their serum levels after exercise stress to those before exercise stress. In the diagram, a represents miR-1; b represents miR-133a; c represents miR-206; and d represents creatine kinase (CK). The open circle/broken line represents B10 mice, and the filled circle/solid line represents mdx mice.

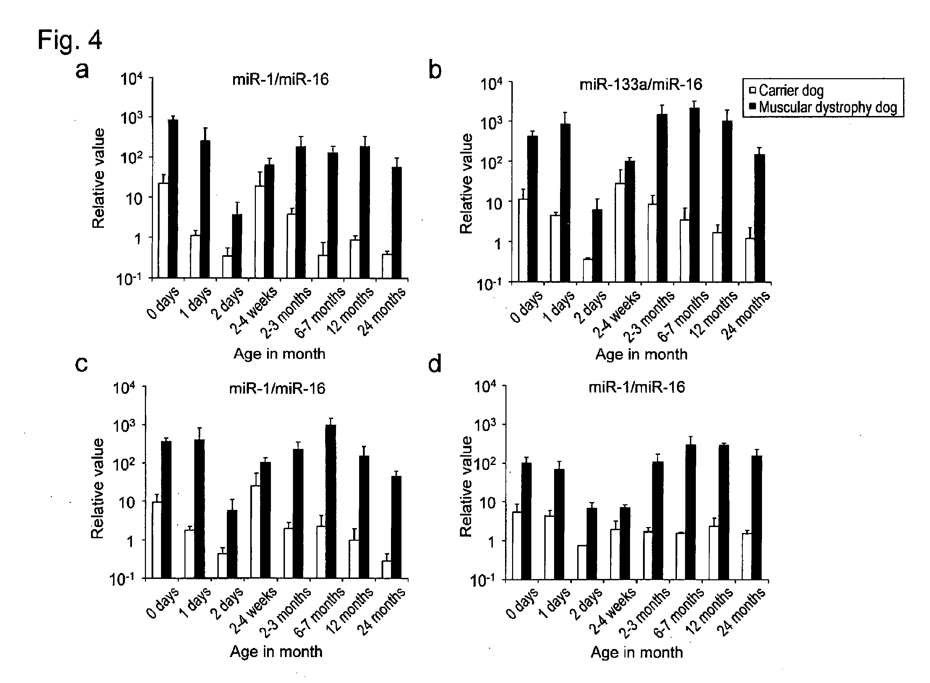

[Figure 4] Figure 4 shows the amount of postnatal change in the serum level of miRNA, etc., in muscular dystrophy dog models CXMDj or carrier dogs thereof. The amounts of miRNA, etc., were corrected with the amount of miR-16 in the serum and then indicated by relative values to the corrected levels of corresponding miRNA, etc., in normal dogs at corresponding ages.

### Description of Embodiments

### 1. Marker for detecting myogenic disease

### 1-1. Summary

The first aspect of the present invention relates to a marker for detecting myogenic disease. The amounts of particular miRNA serving as the marker for detecting myogenic disease of this aspect can be measured in blood to thereby detect the presence or absence of myogenic disease, particularly, muscular dystrophy, affecting a test subject.

### 1-2. Constitution

The marker for detecting myogenic disease of the present invention is constituted by miRNA comprising any of the nucleotide sequences shown in SEQ ID NOs: 1 to 4.

In the present invention, the "marker for detecting myogenic disease" refers to an index for the detection of the presence or absence of myogenic disease affecting a test individual. In the present invention, the marker for detecting myogenic disease corresponds to particular miRNA specifically expressed in skeletal muscles and cardiac muscles, i.e., miR-1, miR-133 (miR-133a and miR-133b), and miR-206 specifically described below. The marker for detecting myogenic disease encompasses all of any one of these miRNAs and any combination of two or more thereof.

In the present invention, the "myogenic disease" refers to a disease that causes muscle wasting or weakness. Examples thereof include muscular dystrophy (including various types of muscular dystrophy such as Duchenne, Becker, Emery-Dreifuss, limb-girdle, facioscapulohumeral, oculopharyngeal, and congenital types). In the present invention, the myogenic disease is preferably muscular dystrophy.

The "miRNA" refers to a single-stranded noncoding RNA of 21 to 23 bases in length present in cells. This small RNA molecule is known to bind to the mRNA of a target gene and protein factors to form a complex called RISC (RNA-induced silencing complex) or miRNP, which in turn acts to regulate the expression of the target gene by inhibiting the translation of the target gene. The miRNA is transcribed from the genome in the state of a precursor (pre-precursor) called pri-miRNA, followed by processing into a precursor called pre-miRNA in the nucleus by endonuclease called Drosha and further converted to mature miRNA by the action of extranuclear endonuclease called Dicer (Bartel DP, 2004, Cell, 116: 281-297). Thus, these precursors pri-miRNA and pre-miRNA and the mature miRNA can usually be found intracellularly. The miRNA of the present invention encompasses all of the miRNA precursors and the mature miRNA. The mature miRNA is preferable. This is because the mature miRNA can directly contribute to the expression regulation of the target gene.

The nucleotide sequence shown in SEQ ID NO: 1 corresponds to human mature miR-1; the nucleotide sequence shown in SEQ ID NO: 2 corresponds to human mature miR-133a; the nucleotide sequence shown in SEQ ID NO: 3 corresponds to human mature miR-133b; and the nucleotide sequence shown in SEQ ID NO: 4 corresponds to human mature miR-206. These miRNAs may correspond to not only human mature miRNA but also mature miRNA of other organism species having the same nucleotide sequence thereas, because their nucleotide sequences are fully conserved in many mammals including mice, rats, and dogs. As described above, it is known that: miR-1 and miR-133 are specifically expressed in cardiac muscles, atria, and skeletal muscles; and miR-206 is specifically expressed in skeletal muscles (Kim et al., 2006, J. Cell Biochem., 174, 677-687).

As described above, the miRNA of the present invention encompasses all of the precursors (pri-miRNA and pre-miRNA) and the mature form. Thus, the marker for detecting myogenic disease of the present invention also encompasses miRNA precursors containing any of the nucleotide sequences shown in SEQ ID NOs: 1 to 4 in a portion of their regions. The marker for detecting myogenic disease of the present invention is more preferably mature miRNAs each of which consists of one of the nucleotide sequences shown in SEQ ID NOs: 1 to 4.

### 1-3. Effect

The marker for detecting myogenic disease of the present invention can be used as a marker for detection in a method for detecting myogenic disease according to the second aspect of the present invention described later.

### 2. Method for detecting myogenic disease

### 2-1. Summary

The second aspect of the present invention relates to a method for detecting the presence or absence of myogenic disease, particularly, muscular dystrophy, affecting a test subject. This method involves measuring the amount of one or more particular miRNA, i.e., the amount of the marker for detecting myogenic disease according to the first aspect, in the blood of the test subject to thereby determine whether or not the test subject has myogenic disease.

### 2-2. Constitution

The detection method of the present invention comprises a measurement step and a comparison step. Hereinafter, each step will be described specifically.

### 2-2-1. Measurement step

The "measurement step" is the step of measuring the amount of the marker for detecting myogenic disease according to the first aspect, i.e., the amount of one or more miRNA(s) comprising any of the nucleotide sequences shown in SEQ ID NOs: 1 to 4, in blood collected from the test subject.

In the present invention, the "test subject" refers to an individual that is subjected to the test of the presence or absence of myogenic disease affecting the individual. An organism that can be used as the test subject is a mammal, preferably a human.

In the present invention, the "blood" encompasses whole blood, plasma, and serum. Any of venous blood, arterial blood, bone marrow fluid, and cord blood may be used. The "blood collected from the test subject" refers to blood that has been collected directly or indirectly from the test subject. Such blood encompasses blood collected from the test subject placed in the resting state before collection and blood collected from the test subject after exercise stress. In this context, the "resting state" refers to the state in which skeletal muscle movement is stopped as much as possible with, for example, a human test subject, lying down or sitting down. Alternatively, the resting state for non-human animals refers to the calm state of life that imparts no excessive exercise or stress thereto. In the present invention, the "exercise stress" refers to a load aggressively applied to skeletal muscles, regardless of the posture of the test subject. The exercise stress corresponds to, for example, walking, running, stepping exercise, or sports.

The directly collected blood encompasses, for example, peripheral blood or bone marrow fluid collected by the direct insertion of an injection needle or the like to the test subject, and, for example, cord blood collected directly from the postpartum umbilical cord. When the blood is collected directly from the test subject, this blood collection may be performed according to a method known in the art. For example, the peripheral blood may be collected by injection to the peripheral vein or the like; the cord blood may be collected by the injection of a needle to the postpartum umbilical cord before placenta delivery; and the bone marrow fluid may be collected by bone marrow aspiration. Peripheral blood collected by injection is more preferable because this collection procedure is low invasive to the test subject and allows easy obtainment at any time.

The indirectly collected blood encompasses, for example, a sample obtained by adding heparin or the like for anticoagulation treatment to the directly collected whole blood or separating plasma or serum therefrom and then temporarily refrigerating or cryopreserving it, followed by recollection therefrom.

Examples of a feature of the present invention include the "blood collected from the test subject" irrespective of the presence or absence of exercise stress to the test subject before blood collection. Creatine kinase previously used in the test of muscular dystrophy, a typical form of myogenic disease, varies in concentration in blood between before and after exercise stress and thus requires placing test subjects under the resting state for blood collection. In the present invention, however, the marker for detecting myogenic disease of the first aspect exhibits a stable concentration in blood before and after exercise stress, as shown in Example 1 described later. Thus, even blood collected from the test subject after exercise stress (e.g., from immediately after stress to 24 hours later) can be used in the present invention.

Usually 50 µL or larger, preferably 100 µL or larger, and 500 µL or smaller, preferably 1 mL smaller suffice as the volume of the blood used in the method of the present invention. The whole blood collected from the test subject is provided in advance with anticoagulation treatment. Examples of methods therefor include a method involving coating in advance, for example, the inside of a syringe for use in blood collection with an anticoagulant such as heparin or a blood coagulation inhibitor, a treatment method involving adding an anticoagulant to collected whole blood (in this case, for example, heparin can be added at a final concentration of 10 to 100 units/mL), a method involving centrifuging the whole blood treated with the anticoagulant or the like at an appropriate speed and preparing the supernatant as plasma, and a method involving leaving whole blood at room temperature, then centrifuging the blood at an appropriate speed, and preparing the supernatant as serum.

The term "amount" according to the present invention represents the quantity of the marker for detecting myogenic disease in blood. Examples thereof include relative amounts such as concentration and the absolute amount of miRNA contained in the predetermined volume of blood. In the present invention, any of relative and absolute amounts may be used. The relative amount is preferable.

In this step, a method for measuring the amount of the marker for detecting myogenic disease of the first aspect in blood is not particularly limited as long as the amount of the marker can be detected and determined by this method. Examples thereof include a nucleic acid amplification method, a hybridization method, and an RNase protection method.

The "nucleic acid amplification method" refers to a method involving amplifying a particular region of a target nucleic acid via nucleic acid polymerase using a forward/reverse primer set. Examples thereof include PCR (including RT-PCR), NASBA, ICAN, and LAMP (registered trademark) (including RT-LAMP). PCR is preferable. This is because: the PCR method is most widely used in the art with rich reagents, kits, reaction equipment, etc.; and various application techniques have been developed. Since the target nucleic acid in the present invention is miRNA, a nucleic acid amplification method mediated by reverse transcription reaction (RT reaction), for example, RT-PCR or RT-LAMP, is typically adopted. Also, since the present invention requires measuring the amount of the marker for detecting myogenic disease in blood, it is preferred to use, particularly, quantitative PCR, for example, real-time PCR, among these nucleic acid amplification methods. The real-time PCR performs analysis by PCR using a thermal cycler equipped with a detector of a fluorescence intensity in a reaction system in which the PCR product is specifically fluorescently labeled during the process of gene amplification reaction. This method is excellent because it can monitor the amount of the product in real time during reaction without the need for sampling and permits computer-assisted regression analysis of the results. Examples of methods for labeling the PCR product include a method using a fluorescently labeled probe, such as TaqMan (registered trademark) PCR, and a method using a reagent specifically binding to double-stranded DNA. The TaqMan PCR method employs a probe modified 5'-terminally with a quencher substance and 3'-terminally with a fluorescent dye. The 5'-terminal quencher substance suppresses the 3'-terminal fluorescent dye in a usual state. Upon PCR, the probe is degraded by the 5' → 3' exonuclease activity of Taq polymerase. As a result, the suppression of the quencher substance is canceled to emit fluorescence. The amount of fluorescence reflects the amount of the PCR product. Since the number of cycles (CT) at which the PCR product reaches the detection limit is in the relationship of inverse correlation with the initial amount of the template, the real-time measurement method determines the initial amount of the template by CT measurement. CT is measured using a series of several known amounts of the template to prepare a calibration curve. The absolute value of the initial amount of the template in an unknown sample can be calculated from the calibration curve. In addition, the amplification product may be detected and quantified in combination with the hybridization method described below.

In this context, the specific method for measuring the amount of miRNA using the nucleic acid amplification method will be described later in detail in the paragraph "2-3. Method".

The "hybridization method" refers to a method involving detecting and quantifying a target nucleic acid or its fragment by use of the base pairing between the nucleic acid and a probe composed of a nucleic acid fragment having a nucleotide sequence completely or partially complementary to the nucleotide sequence of the target nucleic acid to be detected. Some hybridization methods differing in detection means are known. Since the target nucleic acid in the present invention is miRNA, for example, a Northern hybridization method (Northern blotting hybridization method), an RNA microarray method, a surface plasmon resonance method, or a quartz crystal microbalance method is preferable.

The "Northern hybridization method" is the most general analysis method for gene expression, which involves: electrophoretically fractionating RNA prepared from a sample on an agarose or polyamide gel, under denaturation conditions; transferring (blotting) it to a filter; and then detecting target RNA using a probe having a nucleotide sequence specific for the target RNA. The probe may be labeled with an appropriate marker such as a fluorescent dye or a radioisotope to thereby achieve the quantification of the target RNA using a measurement apparatus, for example, a chemiluminescence photographic analyzer (e.g., Light Capture; ATTO Corp.), a scintillation counter, or an imaging analyzer (e.g., FUJIFILM; BAS series). The Northern hybridization method is a technique well known in the art. See, for example, Sambrook, J. et. al., (1989) Molecular Cloning: a Laboratory Manual Second Ed., Cold Spring Harbor Laboratory Press, Cold Spring Harbor, New York; and Current Protocols in Molecular Biology I (1997), jointly translated by Nishino and Sano, Maruzen Co., Ltd.

The "RNA microarray method" refers to a method applying a DNA microarray method to RNA. This method involves: arranging and immobilizing, on a substrate, small spots with a high density of probes each composed of a nucleic acid fragment completely or partially complementary to the nucleotide sequence of a targeted nucleic acid; reacting therewith a sample containing the target nucleic acid; and detecting and quantifying, for example, fluorescently, a nucleic acid hybridized to the substrate spot. The detection and quantification can be achieved by detecting or measuring, for example, fluorescence based on the hybridization of the target nucleic acid or the like using a microplate reader or a scanner. The RNA microarray method is also a technique well known in the art. See, for example, the DNA microarray method (DNA Microarray and Latest PCR Method (2000), Masaaki Muramatsu and Hiroyuki Nawa ed., Gakken Medical Shujunsha Co., Ltd.).

The "surface plasmon resonance (SPR) method" refers to a method involving highly sensitively detecting and quantifying a substance adsorbed on the surface of a thin metal film by use of the so-called surface plasmon resonance phenomenon in which as the thin metal film is irradiated with laser beam at varying angles of incidence, reflected light intensity remarkably attenuates at a particular angle of incidence (resonance angle). In the present invention, for example, a nucleic acid probe having a sequence complementary to the nucleotide sequence of target miRNA is immobilized on the surface of a thin metal film, and the surface portion of the thin metal film other than the miRNA-immobilized region is blocked. Then, blood collected from the test subject is flowed as a sample on the thin metal film surface to thereby form the base pairing between the target miRNA and the nucleic acid probe. The target miRNA can be detected and quantified from the difference in measured value between before and after sample flowing. The detection and quantification by the surface plasmon resonance method can be performed using an SPR sensor commercially available from, for example, Biacore. This technique is well known in the art. See, for example, Kazuhiro Nagata and Hiroshi Handa, Real-Time Analysis of Biomolecular Interactions, Springer-Verlag Tokyo, Inc., Tokyo, Japan, 2000.

The "quartz crystal microbalance (QCM) method" refers to mass spectrometry involving quantitatively monitoring an exceedingly small amount of an adsorbed substance on the basis of the amount of change in resonance frequency by use of the phenomenon in which the resonance frequency of a quartz crystal decreases according to the mass of the substance adsorbed onto the surface of electrodes attached to the quartz crystal. The detection and quantification by this method can employ a commercially available QCM sensor, as in the SPR method. For example, a nucleic acid probe having a sequence complementary to the nucleotide sequence of target miRNA is immobilized on electrode surface and base-paired with the target miRNA in blood collected from the test subject so that the target miRNA can be detected and quantified. This technique is well known in the art. See, for example, J. Christopher Love, L.A. Estroff, J.K. Kriebel, R.G. Nuzzo, G.M. Whitesides (2005) Self-Assembled Monolayers of a Form of Nanotechnology, Chemical Review, 105: 1103-1169; and Toyosaka Moriizumi and Takamichi Nakamoto, (1997), Sensor Engineering, Shokodo Co., Ltd.

The probe used in the hybridization method can be a nucleic acid fragment having a nucleotide sequence completely or partially complementary to any of the nucleotide sequences shown in SEQ ID NOs: 1 to 4. The base length of the probe is 8 bases or more, preferably 10 bases or more, more preferably 12 bases or more, further preferably 15 bases or more, or equal to or less than the full length of the target sequence. Nucleic acids constituting the probe can be usually DNAs, RNAs, or a combination thereof. All or some of these nucleic acids may be chemically modified nucleic acids or pseudo-nucleic acids such as PNA (peptide nucleic acid), LNA (Locked Nucleic Acid; registered trademark), methylphosphonate DNA, phosphorothioate DNA, or 2'-O-methyl RNA, or a combination thereof. In addition, the probe used in the hybridization method can be modified or labeled with, for example, a fluorescent dye (e.g., fluorescamine and its derivatives, rhodamine and its derivatives, FITC, cy3, cy5, FAM, HEX, and VIC), a quencher substance (TAMRA, DABCYL, BHQ-1, BHQ-2, or BHQ-3), biotin or (strept)avidin, a modifying material such as magnetic beads, or a radioisotope (e.g., ³²P, ³³P, and ³⁵S). It is preferred to perform the hybridization under stringent conditions. This is because undesired nucleic acids resulting in nonspecific hybridization are eliminated.

The "RNA protection method" refers to a method for detecting and quantifying target RNA, involving hybridizing the target RNA to a probe having a nucleotide sequence complementary to the target RNA, followed by RNase treatment of the hybridizing sample, electrophoretically separating and detecting hybridized RNA that has escaped degradation. A method for the electrophoretic separation and detection is basically performed in the same way as in the hybridization method.

### 2-2-2. Comparison step

The "comparison step" is the step of relating the statistically significantly higher amount of the miRNA in the blood of the test subject than that of corresponding miRNA in the blood of a normal individual with the presence of myogenic disease affecting the test subject. Whether or not the test subject has myogenic disease is determined by this relation. Specifically, when the amount of any one or more of the particular miRNA in the blood of the test subject is statistically significantly higher than that of corresponding miRNA in the blood of a normal individual, the test subject is confirmed to have myogenic disease or to be likely to develop this disease in the near future.

The "amount of the miRNA in the blood" refers to the quantity of the marker for detecting myogenic disease, i.e., one or more miRNAs comprising any of the nucleotide sequences shown in SEQ ID NOs: 1 to 4, in blood.

In the present invention, the "normal individual" refers to an individual that is of the same species as in the test subject and has been shown to have at least no myogenic disease, preferably, a healthy individual that has been shown to have no disease.

The "corresponding miRNA in a normal individual" refers to miRNA identical to the test subject-derived miRNA measured in the measurement step. For example, when miR-1 in the test subject is subjected to measurement in the measurement step, the corresponding miRNA means miR-1 in a normal individual.

The amount of miRNA in the blood of a normal individual used in this step may be the amount of corresponding miRNA in the blood measured simultaneously with the measurement of the amount of the miRNA in the blood of the test subject in the measurement step of the present invention. Alternatively, the amount of corresponding miRNA in the blood that can be used may be measured in advance under the same conditions as in the measurement of the amount of the miRNA in the blood of the test subject. Thus, the amount of each miRNA in the blood of normal individuals can be measured in advance as a marker for detecting myogenic disease and databased as reference values. This approach is convenient because it does not require measuring the amount of miRNA in the blood of a normal individual in parallel with each measurement of the amount of the marker for detecting myogenic disease in the blood of the test subject.

The phrase "statistically significantly" means that quantitative difference in each miRNA in blood is the significant difference between the test subject and the normal individual in statistical manipulation. Specifically, examples of the phrase "statistically significantly" include the case in which the significance level is smaller than 5%, 1%, or 0.1%. A test method known in the art capable of determining the presence or absence of significance can be used appropriately for testing the statistical manipulation without particular limitations. For example, a student's t test or a multiple comparison test can be used.

The phrase "statistically significantly higher" specifically means that the amount of the marker for detecting myogenic disease in the blood of the test subject is, for example, 5 or more times, preferably 10 or more times, that of the corresponding marker for detecting myogenic disease in the blood of a normal individual. For example, the concentration of miR-1 in the blood of the test subject can exhibit a relative value of 5 or more to that of miR-1 in the blood of a normal individual.

In the present invention, the term "relating" means that comparison results about the amount of the miRNA in the blood, which serves as the marker for detecting myogenic disease, are linked to myogenic disease affecting the test subject or the latent development of this disease. Research by the present inventors has revealed that the amount of the marker for detecting myogenic disease in blood exhibits the statistically significant quantitative difference between an individual having myogenic disease or likely to develop this disease in the near future and a normal individual. In the present invention, based on the findings described above, when the amount of the marker for detecting myogenic disease in the blood of the test subject is statistically significantly higher than that of corresponding miRNA in the blood of a normal individual, the test subject is confirmed to have myogenic disease or to be likely to develop this disease in the near future. The test subject having myogenic disease or likely to develop this disease in the future has significantly higher levels of all miRNAs, particularly, all mature miRNAs, serving as the marker for detecting myogenic disease, than those in a normal individual. Thus, when at least one or more miRNAs constituting the marker for detecting myogenic disease can be related as described above, this test subject can be confirmed to have myogenic disease or to be likely to develop this disease in the near future. For excluding the possibility of false-positive, it is preferred to establish the relation as to two or more miRNAs serving as the marker for detecting myogenic disease.

### 2-3. Method

### <Measurement of amount of miRNA in blood using real-time PCR>

### (1) RNA extraction from blood

When the collected blood is whole blood, serum or plasma can be prepared. For preparing the serum, the whole blood can be left at room temperature for approximately 20 minutes to approximately 1 hour, then cooled on ice, and centrifuged at 2500 rpm to 4000 rpm at 4°C for 10 minutes to 20 minutes to obtain a supernatant. Alternatively, for preparing the plasma, the whole blood can be centrifuged, for example, at 5000 x g at 4°C for 15 minutes.

Any RNA extraction method known in the art may be used for extracting RNA from the blood (whole blood, plasma, serum, and a combination thereof). RNA can be extracted according to the RNA extraction method described in, for example, Sambrook, J. et. al., (1989) Molecular Cloning: a Laboratory Manual Second Ed., Cold Spring Harbor Laboratory Press, Cold Spring Harbor, New York. The RNA can be prepared as total RNA. Also, RNA extraction kits commercially available from various manufacturers may be used. Some of such commercially available RNA extraction kits have been developed for the purpose of efficiently collecting microRNA present in samples and can selectively extract, for example, only small RNA molecules. These kits can also be used preferably in the present invention. Specific examples thereof include mirVana microRNA isolation kit (Ambion). A specific method for using such a kit can be performed according to the protocol included in the kit or a method equivalent thereto.

### (2) Real-time PCR

As described above, the nucleic acid to be detected in the present invention is miRNA whose mature form is only approximately 20 bases in length. Thus, the target nucleic acid is too short to be appropriately amplified by a quantitative nucleic acid amplification method, for example, real-time RT-PCR, mediated by general RT reaction. In this regard, for detecting the marker for detecting myogenic disease in blood using the nucleic acid amplification method, it is preferred to amplify the target nucleic acid using, for example, a kit or special primers commercially available from a manufacturer. One example thereof includes Applied Biosystems TaqMan MicroRNA Assays Kit commercially available from Life Technologies Corp. The Looped RT primer specific for each miRNA attached to the kit is useful because use thereof achieves amplification after efficient reverse transcription of the mature miRNA of interest. The Looped RT primer self-forms a hairpin structure with a 3' overhang of several bases complementary to the sequence of the 3' region of the target mature miRNA. The 3' overhang is annealed with the 3' region of the target miRNA. Then, the target miRNA is elongated as a template by RTase. Then, the elongation product can be used as a template in usual real-time PCR to specifically amplify the target miRNA.

The reaction conditions for real-time PCR are generally based on PCR known in the art. These reaction conditions vary depending on the base length of a nucleic acid fragment to be amplified, the amount of a template nucleic acid, the base lengths and Tm values of primers used, the optimum reaction temperature and optimum pH of nucleic acid polymerase used, etc., and can thus be determined appropriately according to these conditions. As one typical example, approximately 15 to 40 repetitive cycles each involving denaturation reaction performed at 94 to 95°C for 5 seconds to 5 minutes, annealing reaction performed at 50 to 70°C for 10 seconds to 1 minute, and elongation reaction performed at 68 to 72°C for 30 seconds to 3 minutes can be performed, followed by final elongation reaction at 68 to 72°C for 30 seconds to 10 minutes. When the kit commercially available from the manufacturer is used, the method can be performed according to the protocol included in the kit as a rule.

The nucleic acid polymerase used in real-time PCR is DNA polymerase, particularly, heat-stable DNA polymerase. Various types are commercially available as such nucleic acid polymerase and may be used in the present invention. Examples thereof include Taq DNA polymerase attached to the Applied Biosystems TaqMan MicroRNA Assays Kit (Life Technologies Corp.). Particularly, such a commercially available kit is useful because it accompanies a buffer or the like optimized for the activity of DNA polymerase attached thereto.

### 2-4. Effect

The method of this aspect for detecting the presence or absence of myogenic disease affecting a test subject can highly sensitively detect the presence or absence of myogenic disease affecting the test subject without being influenced by exercise stress to the test subject. The conventional methods for detecting myogenic disease affecting a test subject on the basis of creatine kinase levels in blood largely vary in performance due to exercise stress and thus require, for accurate diagnosis, placing test subjects under the resting state before collection of a detection sample such as blood. This, however, imposes excessive exercise limitations on the test subjects and places an enormous burden on the test subjects. Hence, the detection method of this aspect can greatly reduce the burden on the test subjects before blood collection

The method of this aspect for detecting the presence or absence of myogenic disease affecting a test subject permits detection using peripheral blood and is thus low invasive to the test subject during sample collection.

### Examples

### [Example 1]

### <Verification of marker for detecting muscular dystrophy using mouse muscular dystrophy models>

The effects of the marker for detecting muscular dystrophy of the present invention and the method for detecting muscular dystrophy using the same were validated using mouse muscular dystrophy models.

### (Materials)

The mouse muscular dystrophy models used were mdx mice (mdx/B10) (male individuals; 8 weeks old), disease models of Duchenne muscular dystrophy. Duchenne muscular dystrophy is developed by the deficiency of the dystrophin gene caused by X-linked recessive inheritance. Also, B10 mice (male individuals; 8 weeks old) were used as a control (normal individual) group. In this context, the mdx mice have the same genetic background as in the B10 mice except for the deficiency of the dystrophin gene.

### (Method)

### Blood collection and preparation of serum

Each of the mice was brought in an animal experiment facility and then separately caged and preliminarily raised for 1 week or longer to reduce stress given by transportation or environmental change. One week before application of exercise stress to each mouse, 100 µL or more of blood was collected from the tail artery using a 29-G injection needle and a 0.5-mL syringe. Then, each mouse was allowed to run on a treadmill (running machine) at a speed of 5 m/min for 5 minutes then accelerated by 1 m/min every 1 minute for 15 minutes and thereby given exercise stress. Immediately after the completion of exercise stress (within 30 minutes; indicated by 0 h in the graph), 6 hours later, and 48 hours later, blood was collected in the same way as above.

The collected whole blood was left at room temperature for 30 minutes or longer and then centrifuged at 3000 rpm for 10 minutes using a centrifuge (KUBOTA 2410) to obtain a supernatant as serum.

### Determination of creatine kinase activity

Creatine kinase activity in a 50 µL aliquot of each serum was determined using a biochemical analyzer (Fuji Drychem System).

### Measurement of amounts of various miRNA in blood

### • RNA extraction

Total RNA was extracted from the remaining 50 µL aliquot of each serum sample using Ambion mirVana microRNA isolation kit. Finally, RNA was eluted from the column using 50 µL of an eluting solution and used as a total RNA solution. The RNA extraction procedures followed the protocol included in the kit.

### • Quantification of various miRNA by real-time PCR

A 5 µL aliquot of the total RNA solution was used. Each mature miRNA (miR-1: SEQ ID NO: 1, miR-16, miR-132, miR-133a: SEQ ID NO: 2, miR-133b: SEQ ID NO: 3, and miR-206: SEQ ID NO: 4) and mature sno202 (SEQ ID NO: 5) which is one of small nucleolar RNAs (snoRNAs) contained in the eluate were quantified by real-time PCR. Applied Biosystems TaqMan microRNA assay kit (Life Technologies Corp.) was used in the amplification reaction.

Since miR-16 is ubiquitously expressed in sufficient amounts in both B10 and mdx mice and does not differ in expression level therebetween, this miRNA was used as an endogenous control for correcting the amount of total RNA in each sample of this Example. Also, since miR-132 is known to be specifically expressed in neurons, this miRNA was used as a negative control in this Example. The RNA sno202 is generally used as an endogenous control in the quantification of mouse miRNA. The basic procedures followed the protocol included in the kit. Specifically, the quantification was performed as follows:

First, the primers specific for each miRNA or snoRNA used in reverse transcription reaction were Looped RT primers specific for each mature miRNA or mature snoRNA attached to the kit. The mature miRNA or the like has completely the same nucleotide sequence between humans and mice (see miRBase; http://www.mirbase.org/cgi-bin/browse.pl). The reaction reagent used was 15.0 µL in total of a reaction solution consisting of 0.15 µL of 100 mM dNTPs, 1.0 µL of RTase (Superscript), 1.5 µL of 10 × RT buffer, 0.188 µL of RNasin, 3.0 µL of 5 × the primers, and 5.0 µL of 2 µg/µL total RNA. Reaction conditions of reverse transcription involved annealing reaction performed at 16°C for 30 minutes, reverse transcription reaction performed at 42°C for 30 minutes, and RTase inactivation performed at 85°C for 5 minutes. The obtained RT product was then left at 4°C.

Next, nucleic acid amplification reaction was performed under conditions involving 1 cycle of 94°C for 2 minutes and 40 cycles each involving 68°C for 15 seconds and 94°C for 1 minute using a reaction solution made up of 10.0 µl of TaqMan universal PCR master mix (Life Technologies Corp.), 7.5 µl of distilled water, 1.0 µl of 20 × primer set, and 1.5 µl of the RT product. The amplification product was then left at 4°C. The amplification product was detected and quantified using Applied Biosystems 7900HT real-time PCR system (Life Technologies Corp.).

### (1) Amount of miRNA in mouse serum

The average amount of each miRNA or the like in the serum of mdx mice (n=5) without exercise stress, i.e., before exercise was examined on the basis of the results of real-time PCR quantification. When the amount of each miRNA or snoRNA in B10 mouse serum was defined as 1, the amount of the corresponding miRNA or snoRNA in mdx mouse serum was indicated by relative values thereto.

The results are shown in Figure 1. As is evident from the diagram, the relative values of the amounts of miR-16, miR-132, and sno202 in the serum were all approximately 1 and hardly quantitatively differed from those of the normal individuals. By contrast, the amounts of miR-1, miR-133a, miR-133b, and miR-206, which are specifically expressed in skeletal muscles, in the serum were significantly higher than those of the B10 mice. These results demonstrated that miR-1, miR-133a, miR-133b, and miR-206 were able to serve as a marker for detecting muscular dystrophy.

Since miR-133a and its variant miR-133b exhibit almost the same behaviors or functions, only results about miR-133a are shown in Examples below.

### (2) Change in marker for detecting muscular dystrophy after exercise stress (I)

miR-1, miR-133a, and miR-206 selected on the basis of the results of Figure 1 were examined for change in their amounts in the serum caused by exercise stress. The amounts of miR-1, miR-133a, and miR-206 in the serum measured by real-time PCR before exercise and at each point in time elapsing after exercise stress were corrected with the amount of miR-16 (Ct of each miRNA / miR-16 Ct was calculated). Then, the obtained corrected level of each sample was indicated by a relative value to the corrected level (defined as 1) of each marker candidate in B10 mice before exercise.

The results are shown in Figure 2. This diagram shows the relative values of miR-1 (a), miR-133a (b), miR-206 (c), and creatine kinase (CK) (d) in the serum of B10 mice (open circle/broken line) and mdx mice (filled circle/solid line). As is evident from this diagram, the amounts of miR-1, miR-133a, and miR-206 in the serum were higher in mdx mice than in B10 mice. Although the amounts of miR-1, miR-133a, and miR-206 had the same tendency to vary immediately after exercise as in creatine kinase, the amount of this change, i.e., the width of movements in their amounts, was much smaller in these miRNAs than in creatine kinase. For example, the maximum level after exercise stress compared with the level before exercise was 70 or more times in creatine kinase and, by contrast, only 10 or less times in miR-1, miR-133a, and miR-206. These results demonstrated that these miRNAs were able to serve as a marker for detecting muscular dystrophy even after exercise stress.

### (3) Change in marker for detecting muscular dystrophy after exercise stress (II)

miRNAs and creatine kinase were further examined for change in their amounts in the serum caused by exercise stress, as relative values to their respective amounts before exercise. As in the preceding paragraph (2), the amounts of miR-1, miR-133a, and miR-206 in the serum of B10 and mdx mice measured by real-time PCR before exercise and at each point in time elapsing after exercise stress were corrected with the amount of miR-16 (Ct of each miRNA / miR-16 Ct was calculated). Then, the change was examined on the basis of a relative value of the obtained corrected level of each sample after exercise stress to the corresponding corrected level before exercise, i.e., a relative value of the corrected level of each B10 mouse-derived sample after exercise to the corresponding corrected level of B10 mice before exercise or a relative value of the corrected level of each mdx mouse-derived sample after exercise to the corresponding corrected level of mdx mice before exercise.

The results are shown in Figure 3. As is evident from the diagram, mdx creatine kinase exhibited approximately 70-fold increase compared with the value before exercise, whereas increase in the amount of each miRNA was much smaller than that in creatine kinase. These results demonstrated that the amounts of these miRNAs in the serum were hardly susceptible to exercise stress, compared with creatine kinase.

### [Example 2]

### <Verification of marker for detecting muscular dystrophy using dog muscular dystrophy models>

The effects of the marker for detecting muscular dystrophy of the present invention and the detection method using the same were validated using dog muscular dystrophy models. Although mdx mice, unlike human muscular dystrophy patients, do not show symptoms such as gait abnormality, muscular dystrophy dogs deficient in the dystrophin gene by X-linked recessive inheritance as in the mdx mice show symptoms such as gait abnormality similar to those in humans. Thus, effects more similar to those seen in humans can be verified.

### (Materials)

The dog muscular dystrophy models used were beagles of CXMDJ lineage having abnormal dystrophin genes on the X-chromosomes, carrier dogs thereof (female beagles having abnormality in one dystrophin gene on the X chromosomal pair), and normal dogs (beagles free from such abnormality in the dystrophin gene).

### (Method)

100 µL or more of blood was collected from the cutaneous vein in the forelimb or hindlimb of each dog immediately after birth (day 0), 1 day later, 2 days later, 2 to 4 weeks later, 2 to 3 months later, 6 to 7 months later, 12 months later, and 24 months later using a 29-G injection needle and a 0.5-mL syringe. Then, serum was prepared in the same way as in Example 1 and temporarily cryopreserved at -80°C. Three to five preparations were randomly extracted from each group and used in the experiment.

The determination of creatine kinase activity, RNA extraction from the serum, and the real-time PCR quantification of miRNAs (miR-1, miR-16, miR-133a, and miR-206) in the serum using the extracted RNA followed Example 1.

### (Results)

The results are shown in Figure 4. In this diagram, as in Example 1, the levels of miR-1, miR-133a, miR-206, and creatine kinase in the serum of individuals of CXMDJ lineage and carrier dogs quantified by real-time PCR were separately corrected with the quantified level of miR-16 and indicated by relative values of these corrected levels to those of normal individuals of corresponding age in month.

All the amounts of miR-1, miR-133a, and miR-206 in the serum serving as the marker for detecting muscular dystrophy of the present invention exhibited almost the same change as in the behavior of creatine kinase. These results demonstrated that these markers were also effective for dog muscular dystrophy models.

## Claims

1. A method for detecting the presence or absence of myogenic disease affecting a test subject, comprising the steps of:
measuring the amount of one or more miRNAs given as follows :
(i) miRNA comprising the nucleotide sequence shown in SEQ ID NO: 1 in blood collected from the test subject after exercise stress,
(ii) miRNA comprising the nucleotide sequence shown in SEQ ID NO: 2 in blood collected from the test subject,
(iii) miRNA comprising the nucleotide sequence shown in SEQ ID NO: 3 in blood collected from the test subject, and
(iv) miRNA comprising the nucleotide sequence shown in SEQ ID NO: 4 in blood collected from the test subject after exercise stress; and
relating the statistically significantly higher amount of the miRNA in the blood of the test subject than that of corresponding miRNA in the blood of a normal individual with the presence of myogenic disease affecting the test subject.

2. The method according to claim 1, wherein miRNAs comprising the nucleotide sequence shown in SEQ ID NOs: 2 and 3 in the blood are collected from the test subject after exercise stress.

3. The method according to claim 1 or 2, which comprises measuring the amount of two or more miRNAs.

4. The method according to any one of claims 1 to 3, wherein each of the miRNA consists of one of the nucleotide sequences shown in SEQ ID NOs: 1 to 4.

5. The method according to any one of claims 1 to 4, wherein the amount of the miRNA in the blood of the test subject is 5 or more times that of corresponding miRNA in the blood of a normal individual.

6. The method according to any one of claims 1 to 5, wherein the amount of the miRNA in the blood is quantitated by a nucleic acid amplification method or a hybridization method.

7. The method according to claim 6, wherein the nucleic acid amplification method is real-time PCR.

8. The method according to any one of claims 1 to 7, wherein the myogenic disease is muscular dystrophy (including various types of muscular dystrophy such as Duchenne, Becker, Emery-Dreifuss, limb-girdle, facioscapulohumeral, oculopharyngeal, and congenital types).

9. Use of one or more miRNAs given as follows:
(i) miRNA comprising the nucleotide sequence shown in SEQ ID NO: 1 in blood collected from the test subject after exercise stress,
(ii) miRNA comprising the nucleotide sequence shown in SEQ ID NO: 2 in blood collected from the test subject,
(iii) miRNA comprising the nucleotide sequence shown in SEQ ID NO: 3 in blood collected from the test subject, and
(iv) miRNA comprising the nucleotide sequence shown in SEQ ID NO: 4 in blood collected from the test subject after exercise stress, for detecting the presence or absence of myogenic disease affecting said test subject.

10. The use according to claim 9, wherein miRNAs comprising the nucleotide sequence shown in SEQ ID NOs: 2 and 3 in the blood are collected from the test subject after exercise stress.

11. The use according to claim 9 or 10, which comprises two or more miRNAs.

12. The use according to any one of claims 9 to 11, wherein each of the miRNA consists of one of the nucleotide sequences shown in SEQ ID NOs: 1 to 4.

13. The use of any one of claims 9 to 12, wherein said myogenic disease is selected from the group consisting of muscular dystrophy (including various types of muscular dystrophy such as Duchenne, Becker, Emery-Dreifuss, limb-girdle, facioscapulohumeral, oculopharyngeal, and congenital types).

## Patentansprüche

1. Verfahren zum Nachweisen des Vorliegens oder der Abwesenheit einer myogenen Erkrankung, unter der ein Proband leidet, umfassend die Schritte:
Messen der Menge einer oder mehrerer miRNAs, die wie folgt sind:
(i) miRNA, umfassend die Nukleotidsequenz, die in SEQ ID NO: 1 in Blut gezeigt wird, das von dem Probanden nach dem Ausüben einer Belastung entnommen wurde,
(ii) miRNA, umfassend die Nukleotidsequenz, die in SEQ ID NO: 2 in Blut gezeigt wird, das von dem Probanden entnommen wurde,
(iii) miRNA, umfassend die Nukleozidsequenz, die in SEQ ID NO: 3 in Blut gezeigt wird, das von dem Probanden entnommen wurde, und
(iv) miRNA, umfassend die Nukleotidsequenz, die in SEQ ID NO: 4 in Blut gezeigt wird, das von dem Probanden nach dem Ausüben einer Belastung entnommen wurde; und
in Beziehung bringen der statistisch signifikant höheren Menge der miRNA in dem Blut des Probanden als die der entsprechenden miRNA in dem Blut eines normalen Individuums mit dem Vorliegen einer myogenen Erkrankung unter der der Proband leidet.

2. Verfahren nach Anspruch 1, worin miRNAs, die die in SEQ ID NOs: 2 und 3 gezeigte Nukleotidsequenz in dem Blut umfassen, von dem Probanden nach dem Ausüben einer Belastung entnommen werden.

3. Verfahren nach Anspruch 1 oder 2, umfassend das Messen der Menge von 2 oder mehr miRNAs.

4. Verfahren nach einem der Ansprüche 1 bis 3, worin jede der miRNAs aus einer der Nukleotidsequenzen, die in SEQ ID NO: 1 bis 4 gezeigt werden, besteht.

5. Verfahren nach einem der Ansprüche 1 bis 4, worin die Menge der miRNA in dem Blut des Probanden 5 mal oder mehr höher ist als die entsprechende miRNA in dem Blut eines normalen Individuums.

6. Verfahren nach einem der Ansprüche 1 bis 5, worin die Menge der miRNA in dem Blut quantifiziert wird durch ein Nukleinsäureamplifikationsverfahren oder ein Hybridisierungsverfahren.

7. Verfahren nach Anspruch 6, worin das Nukleinsäureamplifikationsverfahren Echtzeit-PCR ist.

8. Verfahren nach einem der Ansprüche 1 bis 7, worin die myogene Erkrankung Muskeldystrophie (einschließlich verschiedene Typen von Muskeldystrophie, wie etwa Duchenne, Becker, Emery-Dreifuss, Glieder-Gürtel, fazioskapulohumerale, oculopharyngeale und kongenitale Typen) ist.

9. Verwendung einer oder mehrerer miRNAs, die wie folgt sind:
(i) miRNA, umfassend die Nukleotidsequenz, die in SEQ ID NO: 1 in Blut gezeigt wird, das von dem Probanden nach dem Ausüben einer Belastung entnommen wurde,
(ii) miRNA, umfassend die Nukleotidsequenz, die in SEQ ID NO: 2 in Blut gezeigt wird, das von dem Probanden entnommen wurde,
(iii) miRNA, umfassend die Nukleotidsequenz, die in SEQ ID NO: 3 in Blut gezeigt wird, das von dem Probanden entnommen wurde, und
(iv) miRNA, umfassend die Nukleotidsequenz, die in SEQ ID NO: 4 in Blut gezeigt wird, das von dem Probanden nach dem Ausüben einer Belastung entnommen wurdezum Nachweis des Vorliegens oder der Abwesenheit einer myogenen Erkrankung unter der dieser Proband leidet.

10. Verwendung nach Anspruch 9, worin die miRNAs, die die Nukleotidsequenz, die in SEQ ID NOs: 2 und 3 in dem Blut umfassen, von dem Probanden nach dem Ausüben einer Belastung entnommen werden.

11. Verwendung nach Anspruch 9 oder 10, umfassend zwei oder mehrere miRNAs.

12. Verwendung nach einem der Ansprüche 9 bis 11, worin die miRNA aus einer der Nukleotidsequenzen, die in SEQ ID NOs: 1 bis 4 gezeigt werden, besteht.

13. Verwendung nach einem der Ansprüche 9 bis 12, worin die myogene Erkrankung ausgewählt wird aus der Gruppe, bestehend aus Muskeldystrophie (einschließlich verschiedene Typen von Muskeldystrophie, wie etwa Duchenne, Becker, Emery-Dreifuss, Glieder-Gürtel, fazioskapulohumerale, oculopharyngeale und kongenitale Typen).

## Revendications

1. Procédé de détection de la présence ou de l'absence d'une maladie myogène affectant un sujet à tester, comprenant les étapes suivantes :
la mesure de la quantité d'un ou de plusieurs miARN donnés comme suit :
(i) un miARN comprenant la séquence nucléotidique représentée par SEQ ID NO : 1 dans le sang prélevé chez un sujet à tester après un stress par exercice,
(ii) un miARN comprenant la séquence nucléotidique représentée par SEQ ID NO : 2 dans le sang prélevé chez le sujet à tester,
(iii) un miARN comprenant la séquence nucléotidique représentée par SEQ ID NO : 3 dans le sang prélevé chez le sujet à tester, et
(iv) un miARN comprenant la séquence nucléotidique représentée par SEQ ID NO : 4 dans le sang prélevé chez le sujet à tester après un stress par exercice ; et
la mise en relation de la quantité statistiquement significativement supérieure du miARN dans le sang du sujet à tester par rapport à celle du miARN correspondant dans le sang d'un individu normal avec la présence d'une maladie myogène affectant le sujet à tester.

2. Procédé selon la revendication 1, dans lequel les miARN comprenant la séquence nucléotidique représentée par SEQ ID NO : 2 et 3 dans le sang sont prélevés chez le sujet à tester après un stress par exercice.

3. Procédé selon la revendication 1 ou 2, qui comprend la mesure de la quantité de deux miARN ou plus.

4. Procédé selon l'une quelconque des revendications 1 à 3, dans lequel chacun des miARN est constitué de l'une des séquences nucléotidiques représentées par SEQ ID NO : 1 à 4.

5. Procédé selon l'une quelconque des revendications 1 à 4, dans lequel la quantité du miARN dans le sang du sujet à tester est 5 fois ou plus celle du miARN correspondant dans le sang d'un individu normal.

6. Procédé selon l'une quelconque des revendications 1 à 5, dans lequel la quantité du miARN dans le sang est quantifiée par un procédé d'amplification d'acide nucléique ou un procédé d'hybridation.

7. Procédé selon la revendication 6, dans lequel le procédé d'amplification d'acide nucléique est la PCR en temps réel.

8. Procédé selon l'une quelconque des revendications 1 à 7, dans lequel la maladie myogène est la dystrophie musculaire (y compris divers types de dystrophie musculaire tels que les types Duchenne, Becker, Emery-Dreifuss, des ceintures, facio-scapulo-huméral, oculopharyngé, et congénital).

9. Utilisation d'un ou de plusieurs miARN donnés comme suit :
(i) un miARN comprenant la séquence nucléotidique représentée par SEQ ID NO : 1 dans le sang prélevé chez un sujet à tester après un stress par exercice,
(ii) un miARN comprenant la séquence nucléotidique représentée par SEQ ID NO : 2 dans le sang prélevé chez le sujet à tester,
(iii) un miARN comprenant la séquence nucléotidique représentée par SEQ ID NO : 3 dans le sang prélevé chez le sujet à tester, et
(iv) un miARN comprenant la séquence nucléotidique représentée par SEQ ID NO : 4 dans le sang prélevé chez le sujet à tester après un stress par exercice, pour la détection de la présence ou de l'absence d'une maladie myogène affectant ledit sujet à tester.

10. Utilisation selon la revendication 9, dans laquelle les miARN comprenant la séquence nucléotidique représentée par SEQ ID NO : 2 et 3 dans le sang sont prélevés chez le sujet à tester après un stress par exercice.

11. Utilisation selon la revendication 9 ou 10, qui comprend deux miARN ou plus.

12. Utilisation selon l'une quelconque des revendications 9 à 11, dans laquelle chacun des miARN est constitué de l'une des séquences nucléotidiques représentées par SEQ ID NO : 1 à 4.

13. Utilisation selon l'une quelconque des revendications 9 à 12, dans laquelle la maladie myogène est choisie dans le groupe constitué de la dystrophie musculaire (y compris divers types de dystrophie musculaire tels que les types Duchenne, Becker, Emery-Dreifuss, des ceintures, facio-scapulo-huméral, oculopharyngé, et congénital).


### SEQUENCE LISTING

<110> National Center of Neurology and Psychiatry
<120> Marker for detection of dystrophy and detection method thereof
<130> PH-4713-PCT
<150> JP 2010-041845
   <151> 2010-02-26
<160> 5
<170> PatentIn version 3.4
<210> 1
   <211> 22
   <212> RNA
   <213> Homo sapiens
<400> 1
   uggaauguaa agaaguaugu au 22
<210> 2
   <211> 22
   <212> RNA
   <213> Homo sapiens
<400> 2
   uuuggucccc uucaaccagc ug 22
<210> 3
   <211> 22
   <212> RNA
   <213> Homo sapiens
<400> 3
   uuuggucccc uucaaccagc ua 22
<210> 4
   <211> 22
   <212> RNA
   <213> Homo sapiens
<400> 4
   uggaauguaa ggaagugugu gg 22
<210> 5
   <211> 22
   <212> RNA
   <213> Homo sapiens
<400> 5
   uagcagcacg uaaauauugg cg 22